(19) Europäisches Patentamt
European Patent Office
Office européen des brevets

(11) Publication number : **0 498 630 A2**

(12) # EUROPEAN PATENT APPLICATION

(21) Application number : **92300956.7**

(22) Date of filing : **04.02.92**

(51) Int. Cl.$^5$ : **C07C 68/06,** C07C 69/96

(30) Priority : **05.02.91 JP 35509/91**

(43) Date of publication of application :
**12.08.92 Bulletin 92/33**

(84) Designated Contracting States :
**DE FR GB**

(71) Applicant : **SHIN-ETSU CHEMICAL CO., LTD.**
**6-1, Ohtemachi 2-chome**
**Chiyoda-ku Tokyo (JP)**

(72) Inventor : **Yagihashi, Fujio**
**No. 28-1-101, Aobadai 1-chome, Midori-ku**
**Yokohama-shi, Kanagawa-ken (JP)**
Inventor : **Furihata, Tomoyoshi**
**No. 2-18, Arima 9-chome, Miyamae-ku**
**Kawasaki-shi, Kanagawa-ken (JP)**
Inventor : **Yamada, Motoyuki**
**No. 259-404, Shimosakunobe, Takatsu-ku**
**Kawasaki-shi, Kanagawa-ken (JP)**
Inventor : **Takamizawa, Minoru**
**No. 8-21, Kitazawa 4-chome**
**Setagaya-ku, Tokyo (JP)**

(74) Representative : **Stoner, Gerard Patrick et al**
**Mewburn Ellis 2 Cursitor Street**
**London EC4A 1BQ (GB)**

(54) **Synthesis of para-tert-butoxycarbonyloxystyrene.**

(57) Para-tert-butoxycarbonyloxystyrene is synthesized by reacting para-acetoxystyrene with an alkali metal alkoxide to form an alkali metal para-vinyl phenolate in a reactor, and tertiary carbonyloxylating the alkali metal para-vinyl phenolate in the same reactor. This process can synthesize the end product in high yields and is thus suitable for large scale commercial manufacture.

EP 0 498 630 A2

This invention relates to a process for synthesizing para-tert-butoxycarbonyloxystyrene which is a suitable monomer for the synthesis of poly(para-tertiary carbonyloxystyrene) which draws attention as a photoresist material of the next generation.

Para-tert-butoxycarbonyloxystyrene is a useful compound as a polymerizable monomer for the synthesis of poly(para-tert-butoxycarbonyloxystyrene) which is one of chemically sensitized photoresist materials expected as promising resist compositions for use in large scale integrated circuit manufacturing process, and for the synthesis of copolymers such as block polymers and block graft polymers containing poly(para-tert-butoxycarbonyloxystyrene) moieties.

The following methods were proposed in the prior art for the synthesis of para-tert-butoxycarbonyloxystyrene.

(1) A first method is, as disclosed in European Patent No. 104250, by converting para-hydroxyacetophenone with potassium tertiary butoxide into a corresponding potassium salt, reacting the salt with di-tert-butyl dicarbonate to form para-tert-butoxycarbonyloxybenzaldehyde, and reacting the intermediate with a Wittig reagent such as methylene triphenylphosphorane according to the following reaction scheme.

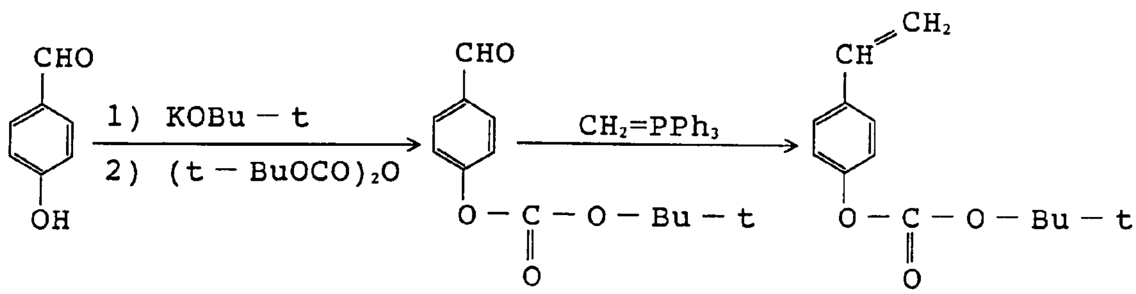


Bu-t and t-Bu each are a tertiary butyl group and Ph is a phenyl group throughout this specification.

(2) A second method is by converting para-hydroxystyrene with potassium tertiary butoxide into a corresponding potassium salt, and reacting the salt with di-tert-butyldicarbonate.

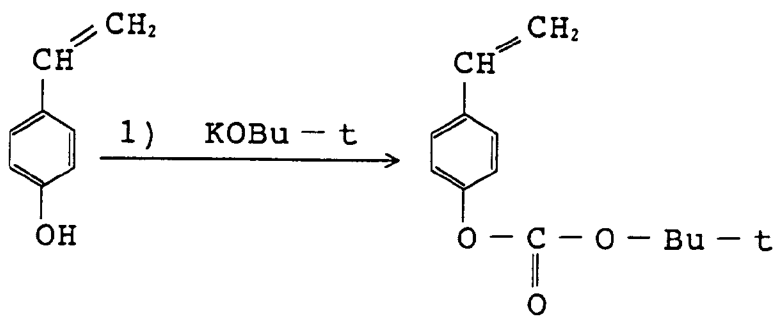


These synthetic methods, however, had the following drawbacks. The first method is cumbersome in operation because it requires several stages of reaction. The intermediate of para-tert-butoxycarbonyloxybenzaldehyde is unstable and remains crystalline at room temperature so that it is difficult to isolate. The second method starts with para-hydroxystyrene which is also crystalline at room temperature and quite likely to polymerize in concentrated state, particularly in crystalline state. Therefore, the second method needs careful handling of the starting material. These methods are not necessarily easy to synthesize para-tert-butoxycarbonyloxystyrene in a mass scale.

A general problem addressed herein is to provide a new way of making para-tert-butoxycarbonyloxystyrene.

More preferably, we seek to provide a commercially advantageous process for synthesizing para-tert-butoxycarbonyloxystyrene from a reactant which is readily available and easy to handle.

The inventors have found that para-tertbutoxycarbonyloxystyrene can be readily synthesized in one pot and in high yields by reacting para-acetoxystyrene, which is not only readily available, but also fully stable and liquid at room temperature and easy to handle, with an alkali metal alkoxide to form an alkali metal para-vinyl

phenolate and tertiary carbonyloxylating the alkali metal para-vinyl phenolate according to the following reaction scheme.

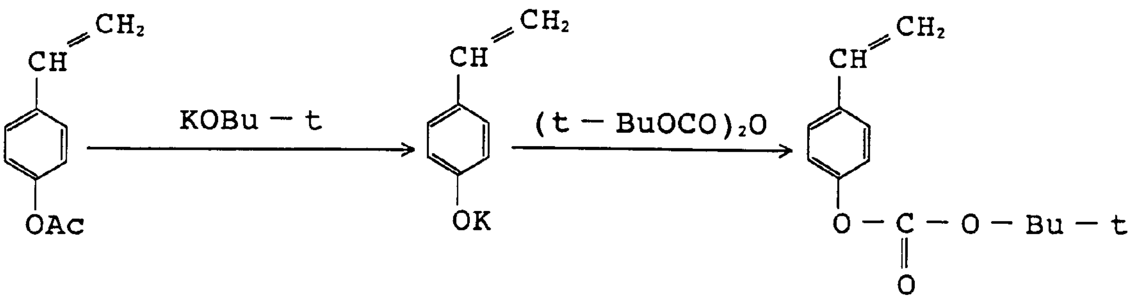


The first step of reaction may be followed by the second step of reaction in the same reactor, and para-tert-butoxycarbonyloxystyrene can be synthesized in a large quantity. Either of these features can lead to significant commercial benefit.

Therefore, the present invention provides a process for synthesizing para-tertbutoxycarbonyloxystyrene comprising the steps of reacting para-acetoxystyrene with an alkali metal alkoxide to form an alkali metal para-vinyl phenolate, and tert-butoxycarbonyloxylating the alkali metal para-vinyl phenolate.

## DETAILED DESCRIPTION

From a chemical reaction aspect, the process of the present invention includes a first stage of reaction of reacting para-acetoxystyrene with an alkali metal alkoxide to form an alkali metal para-vinylphenolate, and a second stage of reaction of tert-butoxycarbonylating the alkali metal para-vinyl phenolate.

The starting reactant is para-acetoxystyrene which is liquid at room temperature, easy to isolate as by distillation, less likely to crystallize or polymerize, and thus quite easy to handle as compared with para-hydroxystyrene or the like.

The alkali metal alkoxides known as alcoholate synthesis reagents include potassium tertiary butoxide, sodium methoxide, and sodium ethoxide, with the potassium tertiary butoxide being preferred.

In the first stage of reaction, para-acetoxystyrene and alkali metal alkoxide are preferably mixed in a molar ratio of from about 1:1 to about 1:2, especially from about 1:1 to about 1:1.2. Reaction would not fully proceed at a molar ratio below the range, and side reaction can take place at a molar ratio beyond the range, both resulting in a lowering of yield.

In the second stage of reaction, the alkali metal para-vinylphenolate resulting from the first stage is subject to tert-butoxycarbonylation. This second stage of reaction can be carried out subsequent to the first stage of reaction in the same reactor.

For tertiary butoxycarbonylation, there may be used reagents commonly used for such purposes, for example, tertiary butyl chloroformate, N-tert-butylcarbonyloxyphthalimide, tert-butylpentachlorophenyl carbonate, 2-(tert-butoxycarbonyloxyimino)-2-phenylacetonitrile, and di-tert-butyl dicarbonate. The tertiary butoxycarbonylation reagent is preferably added in such amounts that the molar ratio of the reagent to the starting reactant or para-acetoxystyrene may range from about 1:1 to about 1:2, especially from about 1:1 to about 1:1.2. Reaction would not fully proceed at a molar ratio below the range, and side reaction can take place at a molar ratio beyond the range, both resulting in a lowering of yield.

Desirably, the second stage of reaction is carried out in an organic solvent. Exemplary organic solvents used include ether solvents such as diethyl ether, dibutyl ether, tetra-hydrofuran, and dioxane; alcohol solvents such as methanol, ethanol, isopropyl alcohol, butanol, and tertiary butanol; and aprotic polar solvents such as dimethylformamide, dimethylsulfoxide, and hexamethylphosphortriamide. The organic solvent is preferably used in an amount of from about 500 to 2,000 ml, especially from about 1,000 to 2,000 ml per mol of the starting reactant.

Reaction conditions for the first and second stages of reaction may be suitably adjusted although the first stage of reaction preferably continues for about 1/2 to 3 hours, especially about 1/2 to 1 hour, and the second stage of reaction preferably continues for about 1/2 to 5 hours, especially about 1/2 to 2 hours, both at a temperature of about 0 to 100°C, especially about 0 to 30°C.

At the end of reaction, the solvent is distilled off in a conventional manner and vacuum distillation will yield the end product, para-tert-butoxycarbonyloxystyrene.

Using synthetic processes as described, we have been able to make para-tert-butoxycarbonyloxystyrene in high yields from a readily available, easy to handle starting reactant through a simple procedure. The process enables mass scale synthesis of para-tert-butoxycarbonyloxystyrene, offering a great commercial benefit.

## EXAMPLE

Examples of the present process are given below by way of illustration and not by way of limitation.

### Example 1

With ice cooling, 310 ml of a 1.0-M solution of potassium tertiary butoxide in tetrahydrofuran was stirred until a temperature of 5°C was reached. To the solution, 50 grams (0.31 mol) of para-acetoxystyrene was added dropwise over 30 minutes. During this addition, the reaction solution was maintained at a temperature below 20°C. At the end of addition, the solution was allowed to stand under the conditions for 30 minutes, obtaining a yellowish orange colored tetrahydrofuran solution of potassium para-vinylphenolate. With stirring and ice water cooling, a solution of 67.28 grams (0.31 mol) of di-tert-butyl dicarbonate in 60 ml of tetrahydrofuran was added dropwise to the reaction solution, which was maintained at a temperature below 20°C. At the end of addition, the reaction mixture was stirred for one hour at room temperature. It was combined with 30 ml of saturated saline water and shaken for subsequent decantation. The organic layer was dried over anhydrous sodium sulfate and the solvent distilled off. The oily residue was subjected to vacuum distillation, yielding 65 grams of a colorless oily product as a fraction of 90 to 92°C /0.2 mmHg. It was para-tert-butoxycarbonyloxystyrene (GC 98.5%, yield 95.7%).

Mass spectrum: m/e

220 ($M^+$), 205 ($M^+$-$CH_3$), 161 ($M^+$-59)
147 ($M^+$-$OBu^t$), 133 ($M^+$-87),
120 ($M^+$-$CO_2Bu^t$, base)

While the invention has been described in what is presently considered to be a preferred embodiment, other variations and modifications will become apparent to those skilled in the art. It is intended, therefore, that the invention not be limited to the illustrative embodiment.

## Claims

1. A process for synthesizing para-tert-butoxycarbonyloxystyrene comprising the steps of:
   reacting para-acetoxystyrene with an alkali metal alkoxide to form an alkali metal para-vinyl phenolate, and
   tertiary carbonyloxylating the alkali metal para-vinyl phenolate.

2. The process of claim 1 wherein the alkali metal alkoxide is potassium tertiary butoxide.

3. A process of claim 1 or claim 2 in which the reaction with alkali metal alkoxide and the carbonyloxylation are carried out in the same reactor vessel.